# EUROPÄISCHE PATENTSCHRIFT

(11) **EP 3 490 516 B1**
(45) Veröffentlichungstag und Bekanntmachung des Hinweises auf die Patenterteilung: **10.03.2021**
(21) Anmeldenummer: 17742225.0
(22) Anmeldetag: 19.07.2017
(51) Int. Cl.: A61H 1/00, A63B 69/00, A63B 69/04, A63B 71/00, A61B 5/11, B25J 9/16, A63B 21/005, A63B 21/00, A63B 24/00, A61B 5/00, A63B 21/008, A63B 21/02

(54) **HIPPOTHERAPIEVORRICHTUNG**
HIPPOTHERAPY DEVICE
DISPOSITIF D'HIPPOTHÉRAPIE

(30) Priorität: 28.07.2016 DE 102016213964
(43) Veröffentlichungstag der Anmeldung: 05.06.2019
(73) Patentinhaber: KUKA Deutschland GmbH, 86165 Augsburg (DE)
(72) Erfinder: KEIBEL, Andreas, 86161 Augsburg (DE)
(74) Vertreter: Oelke, Jochen
(86) Internationale Anmeldenummer: PCT/EP2017/068186
(87) Internationale Veröffentlichungsnummer: WO 2018/019672

(56) Entgegenhaltungen:
- EP-A1- 1 629 868
- WO-A2-2012/119176
- KR-B1- 101 415 462
- US-A1- 2011 054 364
- US-A1- 2014 093 851

## Beschreibung

Die Erfindung betrifft eine Hippotherapievorrichtung, aufweisend einen Sattel, der einen Sitz für eine Person bildet, sowie eine programmierbare Bewegungssteuerung und eine automatische Bewegungsvorrichtung, welche ausgebildet ist, den Sattel nach einem durch die Bewegungssteuerung auszuführenden Bewegungsmusterprogramm, das eine Abfolge an Sollwerten von Positionen und Orientierungen des Sattels im Raum der Bewegungsvorrichtung zur Ausführung der Bewegungsmuster vorgibt, automatisch zu bewegen.

Aus der WO 2012/119176 A2 ist ein Therapieroboter bekannt. Auf einer Basiseinheit mit angeschlossener Steuerung ist dort auf einem Drehgestell ein Roboterarm angebracht, welcher mittels mehrerer Drehgelenke, d.h. Knickgelenke bewegt werden kann. Damit ist es möglich, einen Endeffektor des Therapieroboters, welcher Endeffektor in seiner Form einen Teil des Rückenbereichs eines Pferdes nachbildet, in einem bestimmten Umkreis beliebig im Raum zu positionieren und zu orientieren.

Die KR 101 415 462 B1 offenbart eine Hippotherapievorrichtung nach dem Oberbegriff von Anspruch 1. Insbesondere offenbart diese Druckschrift ein Reitsimulationssystem mit passiver Haltesicherung, um ein herunterfallen des Nutzers zu verhindern.

Die US 2011/0054364 A1 offenbar ebenfalls ein Reitsimulationssystem bei dem der Nutzer durch Benachrichtigungshinweise auf eine fehlerhafte Körperhaltung hingewiesen wird.

Aufgabe der Erfindung ist es, eine Hippotherapievorrichtung zu schaffen, bei der eine die Hippotherapievorrichtung nutzende Person mit Lehr- und/oder Therapieweisen, insbesondere in besonders sicherer Weise, konditioniert und/oder behandelt werden kann.

Die Aufgabe der Erfindung wird durch die Merkmale des Anspruchs 1 gelöst.

Das Bewegungsmusterprogramm kann die für einen Zeitabschnitt vorgesehnen Bewegungen des Sattels automatisch der Bewegungsvorrichtung vorgeben, welche Bewegungen den durch die Therapieweise gewünschten Bewegungen entsprechen. Das Bewegungsmusterprogramm wird insoweit vom Therapeuten der Hippotherapievorrichtung vorgegeben.

Die Erwartungswerte sind Werte von physikalischen Größen, welche die Körperhaltung der Person auf dem Sattel repräsentieren und zwar derjenigen Körperhaltung, die die Person unter sicheren, normalen Bedingungen erwartungsgemäß einnehmen würde oder einnehmen sollte, wie es der momentanen Bewegung des Sattels gemäß dem Bewegungsmusterprogramm entsprechen sollte.

Die Toleranzschwelle definiert Abweichungen der Werte von den Erwartungswerten, die zwar nicht mehr genau der erwarteten Körperhaltung der Person auf dem Sattel entsprechen, aber dennoch in einem Rahmen liegen, der einen weiterhin sicheren Zustand der Person auf dem Sattel kennzeichnen.

Die die Körperhaltung der Person auf dem Sattel kennzeichnende physikalisch Größe kann die räumliche Position und/oder die räumliche Orientierung des Oberkörpers, des Kopfes, wenigstens eines Armes und/oder wenigstens eines Beines der Person sein.

Die auszulösende Sicherungsfunktion kann ein Anhalten der Bewegungsvorrichtung und/oder ein Ausgeben eines den unsicheren Zustand der Person auf dem Sattel kennzeichnenden Signals sein. Ein Anhalten der Bewegungsvorrichtung kann dadurch erfolgen, dass die Motoren oder Antriebe der Bewegungsvorrichtung gestoppt werden und somit der Sattel eine feste Stellung im Raum einnimmt uns sich demgemäß nicht mehr bewegt. Bei einem Anhalten der Bewegungsvorrichtung kann die Person mittels des Körpergurtes und des Grundträgers weiterhin in einer sicheren Stellung gehalten werden. Insbesondere kann bei einem Anhalten der Bewegungsvorrichtung die Person mittels des Körpergurtes und des Grundträgers vor einem Herunterfallen oder Herabrutschen vom Sattel bewahrt werden.

Ein Ausgeben eines den unsicheren Zustand der Person auf dem Sattel kennzeichnenden Signals kann erfolgen, indem die Hilfssteuervorrichtung ein Signal erzeugt und die Hippotherapievorrichtung ein Anzeigemittel aufweist, auf dem das Signal ausgegeben wird. Das Anzeigemittel kann ein Leuchtmittel aufweisen, ein akustisches Ausgabemittel aufweisen, einen Bildschirm aufweisen, auf dem eine den unsicheren Zustand der Person kennzeichnende Information angezeigt wird und/oder eine Sendeeinrichtung und eine Empfangseinrichtung aufweisen. Die Sendeeinrichtung kann dabei eine den unsicheren Zustand der Person kennzeichnende Information senden und die Empfangseinrichtung kann ein Anzeigemittel, einen Bildschirm oder ein akustisches Ausgabemittel, wie einen Lautsprecher aufweisen, an dem die den unsicheren Zustand der Person kennzeichnende Information ausgegeben wird. Die Empfangseinrichtung kann beispielsweise ein Mobiltelefon, ein Pager, oder ein Personenrufempfänger sein. Durch eine den unsicheren Zustand der Person kennzeichnende Informationsausgabe ist eine ständige Anwesenheit eines Therapeuten während des therapeutischen Betriebs der Hippotherapievorrichtung nicht mehr ununterbrochen notwendig. Vielmehr kann die Bewegungssteuerung die Bewegungsvorrichtung automatisch gemäß des auszuführenden Bewegungsmusterprogramms, insbesondere einer Therapievorschrift durchführen, ohne dass ein Therapeut anwesend sein muss. In einem unsicheren Zustand kann jedoch der Therapeut sofort automatisch hinzugerufen werden.

Mit dem Ausgeben eines den unsicheren Zustand der Person auf dem Sattel kennzeichnenden Signals können auch auf akustischem Wege in automatisch gesprochenen Worten oder Sätzen, oder auf optischem Wege in automatisch dargestellten Texten oder Sätzen unmittelbar an die auf dem Sattel sitzende Person Anweisungen automatisch gegeben werden, was die Person tun soll, um den unsicheren Zustand zu beenden oder aufzulösen.

Die auszulösende Sicherungsfunktion ist ein Aktivieren wenigstens eines Aktuators, der auf die räumliche Position und/oder die räumliche Orientierung des Oberkörpers, des Kopfes, wenigstens eines Armes und/oder wenigstens eines Beines der Person einwirkt.

Der wenigstens eine Aktuator kann ausgebildet sein, den am Grundträger verstellbar gelagerten Körpergurt hinsichtlich seiner räumlichen Pose automatisch einzuwirken, wobei die Hilfssteuervorrichtung ausgebildet ist, den wenigstens einen Aktuator in Abhängigkeit der momentanen Stellung und Bewegung des Sattels und der momentanen Körperhaltung der Person auf dem Sattel anzusteuern. Mittels des wenigstens einen Aktuators kann die Person beispielsweise wieder in eine Position, Orientierung und/oder Körperhaltung gebracht werden oder dazu angeregt werden eine Position, Orientierung und/oder Körperhaltung wieder selbst einzunehmen, in der die Person die aufgrund des Bewegungsmusterprogramms erwartete Körperhaltung auf dem Sattel wieder einnimmt oder aufweist.

Der Sattel, welcher einen Reitsitz für eine Person bildet, kann einem echten Reitsattel nachgebildet sein, wie er üblicherweise für das Reiten von Pferden ausgebildet ist. Der Sattel kann jedoch auch auf seine grundlegenden Funktionen reduziert ausgebildet sein. Die grundlegenden Funktionen sind derart, dass der Sattel so ausgebildet sein muss, dass eine Person in zumindest annähernd aufrechter Haltung darauf sitzen kann und die beiden Beine der Person dabei jeweils links und rechts seitlich von dem Sattel, d.h. der Sitzfläche des Sattels herunterhängen können. Optional kann der Sattel ggf. auch über Steigbügel verfügen, an denen sich die Person mit ihren beiden Füßen abstützen kann, wie dies auch beim Reiten von Pferden üblich ist. In einer sehr einfachen Ausführung kann der Sattel jedoch schon allein von einem im Wesentlichen zylindrischen Grundkörper gebildet werden, welcher mit seiner Zylinderachse im Wesentlichen horizontal ausgerichtet angeordnet ist, so dass die Mantelfläche des zylindrischen Grundkörper als eine Sitzfläche für die Person dient. Die beiden Beine der Person hängen dann links und rechts seitlich am zylindrischen Grundkörper herunter.

Die programmierbare Bewegungssteuerung kann einen Roboter, insbesondere einen Industrieroboter aufweisen, an dessen Roboterflansch als ein Endeffektor der Sattel angeordnet ist. Die programmierbare Bewegungssteuerung kann demgemäß einen Roboterarm und eine programmierbare Robotersteuerung umfassen.

Roboterarme mit zugehörigen programmierbaren Robotersteuerungen, insbesondere Industrieroboter sind Arbeitsmaschinen, die zur automatischen Handhabung ausgerüstet werden können und in mehreren Bewegungsachsen beispielsweise hinsichtlich Orientierung, Position und Arbeitsablauf programmierbar sind. Industrieroboter weisen üblicherweise einen Roboterarm mit mehreren über Gelenke verbundene Glieder und programmierbare Robotersteuerungen (Steuervorrichtungen) auf, die während des Betriebs die Bewegungsabläufe des Roboterarms automatisch steuern bzw. regeln, um einen Roboterflansch des Roboterarms im Raum zu positionieren und zu bewegen. Die Glieder werden dazu über Antriebsmotoren, insbesondere elektrische Antriebsmotoren, die von der Robotersteuerung angesteuert werden, insbesondere bezüglich der Bewegungsachsen des Industrieroboters, welche die Bewegungsfreiheitsgrade der Gelenke repräsentieren, bewegt. Der Roboter kann beispielsweise ein Industrieroboter sein, der insbesondere ein Knickarmroboter mit seriell aufeinander folgenden Drehachsen, beispielsweise fünf, sechs oder sieben Drehachsen sein kann. Der Sattel der Hippotherapievorrichtung kann demgemäß dadurch im Raum bewegt werden, dass die Gelenke des Roboterarms angesteuert durch die programmierbare Robotersteuerung bewegt, d.h. verstellt werden.

Das vorgegebene Bewegungsmuster, welches Reitbewegungen simuliert, kann in der programmierbaren Bewegungssteuerung, insbesondere in der programmierbaren Robotersteuerung gespeichert und von dort zur Ausführung abrufbar sein. Das vorgegebene Bewegungsmuster wird ausgeführt, indem die programmierbare Bewegungssteuerung den Sattel durch Verstellen der Gelenke bspw. des Roboterarms im Raum bewegt, um Reitbewegungen zu simulieren.

Die Personensicherungseinrichtung ist eine von der Bewegungssteuerung und von dem Sattel unterschiedliche Vorrichtung, welche ausgebildet ist, nicht den Sitz, d.h. den Beckenbereich der Person zu kontrollieren und/oder zu halten, sondern ausgebildet ist, den Oberkörper der Person zu kontrollieren und/oder zu halten. Demgemäß kann die Personensicherungseinrichtung einen Körpergurt aufweisen, der den Grundträger der Personensicherungseinrichtung mit dem Oberkörper der Person verbindet. Dabei ist der Körpergurt verstellbar an dem Grundträger gelagert. Der Körpergurt kann in Art eines Klettergurtes oder eines Rettungsgurtes ausgebildet sein. Der Körpergurt kann insbesondere wenigstens einen die Person umschließenden Brustgurt, Bauchgurt und/oder Beckengurt aufweisen. Der Körpergurt kann beispielsweise ein Brustgeschirr aufweisen. Auch wenn die Personensicherungseinrichtung eine von der Bewegungssteuerung und von dem Sattel unterschiedliche Vorrichtung ist, kann die Personensicherungseinrichtung wahlweise entweder als eigenständige Vorrichtung auf einem Boden oder Fundament aufgestellt sein, oder als eine angebrachte Zusatzvorrichtung an der Bewegungssteuerung oder an dem Sattel befestigt sein.

Die Personensicherungseinrichtung ist insoweit eine von der Bewegungssteuerung und von dem Sattel unterschiedliche Vorrichtung, als die Personensicherungseinrichtung wenigstens einen eigenen Aktuator aufweist, der separat von den Antrieben der automatisch verstellbaren Gelenke der Bewegungssteuerung ist. Der wenigstens eine Aktuator kann ein aktiver Aktuator sein. Der aktive Aktuator kann beispielsweise ein Antrieb oder ein Motor sein. Der wenigstens eine Aktuator kann aber auch ein passiver Aktuator sein. Der passive Aktuator kann beispielsweise eine Bremse oder ein Schwingungsdämpfer sein, der eine bereits auf die Personensicherungseinrichtung aufgeprägte Bewegung beeinflusst, d.h. verändert.

Unter einem Aktuator wird im Rahmen der Erfindung demgemäß ganz allgemein ein Bauelement (oder eine Baugruppe) verstanden, das insbesondere elektrische Signale in mechanische Bewegung oder in andere physikalische Größen umsetzen kann. Die mechanische Bewegung kann eine Bewegung mit konstanter Geschwindigkeit sein. Die mechanische Bewegung kann jedoch insbesondere auch eine Beschleunigung oder ein Abbremsen, also eine negative Beschleunigung sein. Ein Umsetzen in eine andere physikalische Größe kann beispielsweise, wie im Rahmen der Erfindung beschrieben, ein Dämpfen einer Schwingungsbewegung sein. Unter einem Aktuator wird somit ein Aktor, d.h. ein Antriebselement verstanden, das passiv und/oder aktiv die Personensicherungseinrichtung beeinflusst.

Die Hilfssteuervorrichtung steuert den wenigstens einen Aktuator an, entweder um den am Grundträger gelagerten Körpergurt aktiv, d.h. mittels eines Antriebs oder eines Motors zu bewegen und/oder passiv durch Bremsen und/oder Dämpfen einer vorhandenen Bewegung des Körpergurts bezüglich des Grundträgers abzubremsen und/oder zu dämpfen.

Die Hilfssteuervorrichtung kann mit der Bewegungssteuerung, insbesondere mit einer programmierbaren Robotersteuerung kommunizieren.

Erfindungsgemäß kann die Person oder ein Patient auf dem Sattel der Hippotherapievorrichtung, die ein Hippotherapiesystem bilden kann, Platz nehmen. Nach Einrichtung eines Bewegungsmusters beispielsweise eine Therapie mit Hilfe eines Therapeuten, kann ohne weitere Anwesenheit des Therapeuten die Therapie durchgeführt werden.

Dabei wird die Person mit der Personenhalteinrichtung auf dem Sattel gesichert, so dass die Person zunächst nicht herunter fallen kann. Diese kann in verschiedenen Ausprägungen umgesetzt werden. Beispielsweise kann die Person oder der Patient zusammen mit dem Therapeuten den Therapieplatz erreichen. Der Therapeut hilft dem Patienten auf den Sattel. Danach sichert der Therapeut den Patienten mittels der Personenhalteinrichtung gegen Herunterrutschen und/oder Herunterfallen. Der Therapeut richtet anschließend die Therapie ein bzw. wählt ein vorhandenes Bewegungsmuster zur Anwendung aus und startet den Therapievorgang.

Die Personenhalteinrichtung kann insbesondere gegen das Herunterfallen des Patienten vom Therapiesitz die folgenden Eigenschaften aufweisen. Da der Patient während der Therapie bewegt wird, darf die Personenhalteinrichtung, d.h. der Körpergurt nicht rigide und raumfest ausgelegt sein, sondern muss der Person bzw. dem Patienten zumindest annähernd kraftlos folgen.

Sofern der Patient seine Therapieposition zu verlieren droht, also etwa vom Sattel rutscht, kann die Halteeinrichtung aktiv werden und das weitere Herunterrutschen verhindern, ohne dass dem Patienten dadurch ein Schmerz oder anderer Schäden oder ein anderes Risiko entsteht.

Sofern der Patient nach einer ggf. einstellbaren Zeit nicht wieder in eine aufrechte Position zurückkehrt, so kann die Personenhalteinrichtung beispielsweise einen Alarm auslösen und einen Therapeuten ordern, damit dieser den ordnungsgemäßen Zustand wieder herstellen kann.

Ein solches Sicherheits- und Haltesystem kann mit Sensoren ausgestattet sein, welche die Patientenhaltung auf dem Sattel aktiv und sicher überwachen kann. Diese Überwachung kann in mehreren Stufen und mit Hilfe verschiedener gemessener Indizien erfolgen.

Ein Gurtsystem, das von oben den Patienten am Oberkörper hält, kann die Zugkraft und/oder Wegstrecke messen, die der Patient auf die Halterung ausübt bzw. die der Oberkörper von der aufrechten Haltung entfernt ist. In die Gurte eingebaute Zugkraft-Sensoren, wie beispielsweise Wägezellen, können eine Asymmetrie und/oder den Belastungszustand erkennen, der zu weiterführenden, haltungskorrigierenden Bewegungen der Bewegungsvorrichtung, insbesondere des Roboterarms führen kann. Der Roboter kann automatisch eine Korrekturbewegung ausführen, um den Patienten aufzurichten, um ihn daran zu erinnern, gerade zu sitzen. Wenn das keinen Erfolg hat, dann kann die Therapie auch abgebrochen und automatisch eine Therapeutenanforderung abgesetzt werden.

Die Patientensicherung, die insbesondere den Oberkörper umschließt, kann gegebenenfalls die Neigung der Schultern und der Wirbelsäule beispielsweise mittels Neigungssensoren messen, so dass ein drohendes Herunterrutschen früh erkannt werden kann.

Die Hippotherapievorrichtung kann verschiedene intelligente Verhaltensweisen realisiert, mit denen ein Abrutschen der Person vom Sattel verhindert werden kann, so dass der Patient nicht mit seinem gesamten Gewicht in die Halteeinrichtung hineinrutscht. Bei Erkennung von einem Haltungsfehler wird z.B. die Therapiebewegung erst langsamer und dann ganz unterbrochen. Gegebenenfalls wird auch eine besondere Bewegung des Sattels aktiviert, mit der der Patient entweder an eine gerades Sitzen erinnert wird, oder er mechanisch aufgerichtet wird.

Sofern sich der Zustand dann nicht verbessert, in dem der Patient selbst versucht, die korrekte Haltung wieder einzunehmen, kann die Personensicherungseinrichtung beispielsweise motorisch aktiv gegensteuern und eine Bewegung ausführen, mit welcher versucht wird, den Patienten wieder aufzurichten, im Falle einer aktuierten Halteeinrichtung. Diese Therapieunterbrechung bewirkt beim Patienten eine aktive Wahrnehmung seines Haltungsproblems.

Sollte sich der Zustand nicht verbessern, kann die Therapie unterbrochen werden und beispielsweise ein Anforderungs-Signal für den Therapeuten gesetzt werden, so dass dieser den Vorgang übernehmen kann und entscheiden kann, ob die Therapie und falls ja, wie die Therapie weiter gehen kann.

Die Bewegungssteuerung, insbesondere der Roboter bzw. das mechatronische System mit dem der Sattel bzw. der Sitz der Person oder des Patienten bewegt wird, kann insbesondere folgende Sicherheitseinrichtungen aufweisen, die mindestens zweikanalige ausgeführt sein können.

Die Sicherheitseinrichtungen können umfassen einen in sicherer Technik ausgeführten, kartesisch überwachten Arbeitsraum (Einstieg, Therapie-Höhe, -Breite, -Tiefe), eine sichere kartesische Geschwindigkeit und Beschleunigung, einen sicher überwachten Arbeitsraum auf Achsebene, eine sicher überwachte Geschwindigkeit auf Achsebene.

Im Folgenden sind einige Ausführungsvarianten näher erläutert.

Der Grundträger kann beispielsweise an einem Fundament befestigt sein, an dem auch die programmierbare Bewegungsvorrichtung, insbesondere ein Grundgestell eines Roboterarms, befestigt ist. Die verstellbare Lagerung des Körpergurts erlaubt dann eine entsprechende Anzahl von Freiheitsgraden, d.h. eine entsprechende Anzahl von Beweglichkeiten in wenigstens einer Richtung und/oder wenigstens einer Orientierung im Raum relativ zum Fundament, insbesondere relativ zur programmierbaren Bewegungsvorrichtung oder relativ zum Grundgestell des Roboterarms.

Der Grundträger kann aber auch an einem verstellbaren Glied der programmierbaren Bewegungsvorrichtung, insbesondere an einem Teil eines Roboterarms oder an dem Sattel befestigt sein, der durch die programmierbare Bewegungsvorrichtung bewegt wird. Die verstellbare Lagerung des Körpergurts erlaubt dann eine Anzahl von Freiheitsgraden, d.h. eine Anzahl von Beweglichkeiten in wenigstens einer Richtung und/oder wenigstens einer Orientierung im Raum relativ zum Sattel oder relativ zum Flansch eines Roboterarms.

Die Richtungen im Raum können durch ein, zwei oder drei kartesische Koordinatenrichtungen gekennzeichnet sein und/oder die ein, zwei oder drei Orientierungen im Raum können die Drehungen jeweils um eine der drei kartesischen Koordinatenrichtungen sein.

In einer Ausführungsvariante kann der Körpergurt mittels einer Lagervorrichtung oder eines Lagers in wenigstens einer Richtung und/oder wenigstens einer Orientierung im Raum am Grundträger verstellbar gelagert sein und das Lager kann dabei eine Bremsvorrichtung aufweisen, die ausgebildet ist, eine Bewegung des Lagers mit wahlweise unterschiedlichen Bremseigenschaften zu bremsen, die mittels des Aktuators einstellbar sind und zwar angesteuert durch die Hilfssteuervorrichtung. Der Aktuator kann demgemäß die Bremsvorrichtung beeinflussen, insbesondere ansteuern oder der Aktuator kann die Bremsvorrichtung selbst sein.

Die Bremsvorrichtung kann hinsichtlich ihrer Bremswirkung automatisch veränderbar ausgebildet sein. D.h. die Höhe der Bremswirkung kann durch die Hilfssteuervorrichtung automatisch verstellbar sein. Insbesondere kann die Hilfssteuervorrichtung die Höhe der Bremswirkung im zeitlichen Verlauf automatisch verändern und zwar in Abhängigkeit der momentanen Position und/oder Orientierung des Körpergurtes.

Die Bremsvorrichtung kann der Lagervorrichtung bzw. dem Lager zugeordnet sein. Das Lager kann ein Drehlager und/oder ein Schiebelager sein. Die Bremsvorrichtung kann beispielsweise Bremsbeläge aufweisen, die automatisch verstellbar sind und mit unterschiedlich hohen Normalkräften gegen eine Bremsscheibe gedrückt werden können. Die Bremsvorrichtung kann eingerichtet sein, zumindest oder lediglich eine Verstellung des Lagers aufgrund von Schwerkrafteinflüssen zu bremsen. Alternativ oder ergänzend kann die Bremseinrichtung auch eingerichtet sein, ein angetrieben in Bewegung versetztes Lager abzubremsen.

In einer anderen, alternativen oder ergänzenden Ausführungsvariante kann der Körpergurt mittels einer Lagervorrichtung oder eines Lagers in wenigstens einer Richtung und/oder wenigstens einer Orientierung im Raum am Grundträger schwingend gelagert sein und das Lager kann dabei eine Schwingungsdämpfungsvorrichtung aufweisen, die ausgebildet ist, eine Bewegung des Lagers mit wahlweise unterschiedlichen Schwingungsdämpfungseigenschaften zu dämpfen, die mittels des Aktuators einstellbar sind und zwar angesteuert durch die Hilfssteuervorrichtung. Der Aktuator kann demgemäß die Schwingungsdämpfungsvorrichtung beeinflusse, insbesondere ansteuern, oder der Aktuator kann die Schwingungsdämpfungsvorrichtung selbst sein.

Die Lagerung oder das Lager kann dabei beispielsweise durch eine Federvorrichtung gebildet werden. Die Federvorrichtung kann beispielsweise eine Federwendel mit einer festen Federsteifigkeit aufweisen. Die Federvorrichtung kann aber auch beispielsweise eine Öl- oder Gasdruckfeder aufweisen, deren Federsteifigkeit einstellbar sein kann. So kann der Aktuator beispielsweise eine Drosselvorrichtung der Öl- oder Gasdruckfeder ansteuern oder diese Drosselvorrichtung sein.

Die Schwingungsdämpfungsvorrichtung kann mit der Bremsvorrichtung kombiniert sein.

In einer weiteren, alternativen oder ergänzenden Ausführungsvariante kann der Körpergurt mittels einer Antriebsvorrichtung in wenigstens einer Richtung und/oder wenigstens einer Orientierung im Raum am Grundträger angetrieben gelagert sein und die Antriebsvorrichtung kann dabei wenigstens einen Motor aufweisen, der den Aktuator bildet und der ausgebildet ist, den Körpergurt automatisch angetrieben zu bewegen und zwar angesteuert durch die Hilfssteuervorrichtung.

Die Antriebsvorrichtung kann wenigstens einen Motor aufweisen, der eingerichtet ist, die Lagervorrichtung bzw. das Lager automatisch zu verstellen. Der Aktuator kann demgemäß die Antriebsvorrichtung bzw. den wenigstens einen Motor beeinflusse, insbesondere ansteuern, oder der Aktuator kann die Antriebsvorrichtung bzw. der wenigstens eine Motor selbst sein. So kann beispielsweise der wenigstens eine Motor durch die Hilfssteuervorrichtung angetrieben, insbesondere in unterschiedlichen Drehgeschwindigkeiten und Drehrichtungen angetrieben und insbesondere auch abgebremst werden.

Die Antriebsvorrichtung kann mit der Bremsvorrichtung und/oder mit der Schwingungsdämpfungsvorrichtung kombiniert sein.

In allen Ausführungsvarianten kann die Hippotherapievorrichtung wenigstens einen Sensor aufweisen, der ausgebildet ist, die räumliche Position und/oder die räumliche Orientierung des Körpers der Person zu erfassen.

Der Sensor muss dabei nicht notwendiger Weise unmittelbar am Körper der Person oder am Körpergurt befestigt sein. Vielmehr kann der Sensor auch getrennt von dem Körper der Person und getrennt vom Körpergurt separat angeordnet sein und die räumliche Position und/oder die räumliche Orientierung des Körpers der Person aus der Ferne erfassen. So kann der Sensor beispielsweise von einem optischen Sensor, wie einer Kamera gebildet werden, welche die räumliche Position und/oder die räumliche Orientierung des Körpers der Person optisch erfasst, zugeordneten Bilddaten erzeugt, wobei die Bilddaten ausgewertet werden können, um die momentane räumliche Position und/oder die räumliche Orientierung des Körpers der Person numerisch zu bestimmen.

In einer speziellen Ausführungsform kann jedoch der Körpergurt den wenigstens einen Sensor aufweisen. In dieser speziellen Ausführungsform kann der Sensor dann beispielsweise mindestens eine Lagesensor und/oder einen Beschleunigungssensor aufweisen, der ausgebildet ist, die momentane Lage des Körpergurts und/oder die momentane Bewegung des Körpergurts zu erfassen. Aus der momentanen Lage des Körpergurts und/oder die momentane Bewegung des Körpergurts, insbesondere über einen zeitlichen Verlauf der Lage des Körpergurts und/oder die Bewegung des Körpergurts kann dann auf die genaue Position und Lage des Körpers der Person geschlossen werden.

Generell kann der wenigstens eine Sensor über eine Kommunikationsverbindung mit der Hilfssteuervorrichtung verbunden sein, derart, dass Informationen über die momentane Position und/oder momentane Orientierung des Körpers der Person im Raum, an die Hilfssteuervorrichtung übertragen wird, um die momentane Körperhaltung der Person mittels der Hilfssteuervorrichtung oder der Bewegungssteuerung automatisch zu bestimmen.

Der wenigstens eine Sensor kann demgemäß kabelgebunden oder drahtlos mit der Hilfssteuervorrichtung kommunizieren. Eine Kommunikation kann insoweit schon allein durch ein Übermitteln oder Senden von Messwerten des wenigstens einen Sensors an die Hilfssteuervorrichtung gegeben sein. Der wenigstens eine Sensor muss nicht notweniger Weise auch zum empfangen von Informationen oder Daten von der Hilfssteuervorrichtung eingerichtet sein. Der wenigstens eine Sensor kann auch einfach auf analoger Weise mit der Hilfssteuervorrichtung elektrisch verbunden sein.

In einer ersten Ausführungsform kann der Körpergurt mittels eines Tragseiles am Grundträger aufgehängt gelagert sein und die Hippotherapievorrichtung dabei einen Winkelaufnehmer aufweisen, der ausgebildet ist, die Winkelstellung des Tragseiles zu erfassen. Die Winkelstellung des Tragseiles kann dabei eine erste Winkelkomponente aufweisen, welche den Auslenkungswinkel des Tragseils relativ zur senkrechten Richtung definiert und eine zweite Winkelkomponente aufweisen, welche die Richtung der Auslenkung des Tragseils definiert. Das Tragseil kann eine feste Tragseillänge aufweisen. Alternativ kann das Tragseil längenveränderlich ausgebildet sein.

In einer zweiten, alternativen oder ergänzenden Ausführungsform kann der Körpergurt demgemäß mittels eines längenveränderlichen Tragseiles am Grundträger aufgehängt gelagert sein und die Hippotherapievorrichtung dabei einen Längenaufnehmer aufweisen, der ausgebildet ist, die momentane Länge des Tragseiles zu erfassen.

Das längenveränderliche Tragseil kann beispielsweise eine Seilrolle umfassen, auf der eine erste Teillänge des Tragseils aufgerollt gespeichert ist. Eine zweite Teillänge des Tragseils, die nicht auf der Seilrolle aufgerollt gespeichert ist, bildet eine freie, veränderliche Seillänge, deren momentane Länge mittels des Längenaufnehmers erfasst werden kann.

In einer dritten, alternativen oder ergänzenden Ausführungsform kann der Körpergurt mittels eines Tragseiles am Grundträger aufgehängt gelagert sein und die Hippotherapievorrichtung dabei einen Kraftaufnehmer aufweisen, der ausgebildet ist, die Zugkraft am Tragseil zu erfassen.

Der Kraftaufnehmer kann insbesondere an der Personensicherungseinrichtung angeordnet sein. Beispielsweise kann der Kraftaufnehmer am Grundträger befestigt und an das Tragseil angekoppelt sein.

In einer besonderen Ausführungsform, die alternativ oder ergänzend zu anderen Ausführungsarten vorhanden sein kann, kann der Körpergurt wenigstens einen ersten Traggurtabschnitt aufweisen, der einen Körpergurtabschnitt des Körpergurts an einer ersten Verbindungsstelle des Körpergurtabschnitts mit einer Anschlussstelle des Tragseils verbindet und der Körpergurt wenigstens einen zweiten Traggurtabschnitt aufweist, der den Körpergurtabschnitt an einer von der ersten Verbindungsstelle verschiedenen, zweiten Verbindungsstelle des Körpergurtabschnitts mit der Anschlussstelle des Tragseils verbindet, wobei der erste Traggurtabschnitt einen ersten Aufnehmer aufweist, der wenigstens eine physikalische Größe an dem ersten Traggurtabschnitt erfasst und der zweite Traggurtabschnitt einen zweiten Aufnehmer aufweist, der wenigstens eine physikalische Größe an dem zweiten Traggurtabschnitt erfasst. Die wenigstens eine physikalische Größe kann eine Kraft, ein Moment, eine räumliche Position/Orientierung, eine Geschwindigkeit und/oder eine Beschleunigung sein.

Die erste Verbindungsstelle und die zweite Verbindungsstelle können in einem Abstand voneinander angeordnet sein und zwar bei aufrechter Körperhaltung, d.h. bei einem symmetrisch hängenden Tragegurt, auf derselben Höhe am Tragegurt. Beispielsweise können die erste Verbindungsstelle und die zweite Verbindungsstelle in Nähe der beiden Schultern der Person am Tragegurt angeordnet sein. Indem der erste Traggurtabschnitt einen ersten Aufnehmer aufweist, der wenigstens eine physikalische Größe an dem ersten Traggurtabschnitt erfasst und der zweite Traggurtabschnitt einen zweiten Aufnehmer aufweist, der wenigstens eine physikalische Größe an dem zweiten Traggurtabschnitt erfasst, können der erste Aufnehmer und der zweite Aufnehmer aus den erfassten physikalischen Größen, insbesondere aus der Abweichung der beiden durch den ersten Aufnehmer und den zweiten Aufnehmer gemessenen Werte feststellen, ob die Person in einer aufrechten Körperhaltung ist oder schief sitzt, bzw. droht von dem Sattel herunterzurutschen.

In allen Ausführungsarten kann die Bewegungssteuerung eingerichtet sein, das vorgegebene Bewegungsmuster zur Ansteuerung der Bewegungsvorrichtung, in Abhängigkeit des Einwirkens des wenigstens einen Aktuators auf den am Grundträger verstellbar gelagerten Körpergurt, automatisch abzuändern.

Verschiedene Ausführungsbeispiele der Erfindung sind exemplarisch in den beigefügten schematischen Zeichnungen dargestellt. Konkrete Merkmale dieser Ausführungsbeispiele können unabhängig davon, in welchem konkreten Zusammenhang sie erwähnt sind, gegebenenfalls auch einzeln oder in anderen als den dargestellten Kombinationen betrachtet, allgemeine Merkmale der Erfindung darstellen.

Es zeigen:
- Fig. 1: eine schematische Darstellung von der Seite einer ersten Ausführungsform einer erfindungsgemäßen Hip-potherapievorrichtung mit einer stationären Personensicherungseinrichtung,
- Fig. 2: eine schematische Darstellung von der Seite einer zweiten Ausführungsform einer erfindungsgemäßen Hip-potherapievorrichtung mit einer Personensicherungseinrichtung, die an einer Bewegungsvorrichtung der Hippotherapievorrichtung befestigt ist, und
- Fig. 3: eine schematische Darstellung von Vorne auf eine Hippotherapievorrichtung gemäß Fig.1 oder Fig.2 mit Sensoren, die an einem Körpergurt der Personensicherungseinrichtung angeordnet sind.

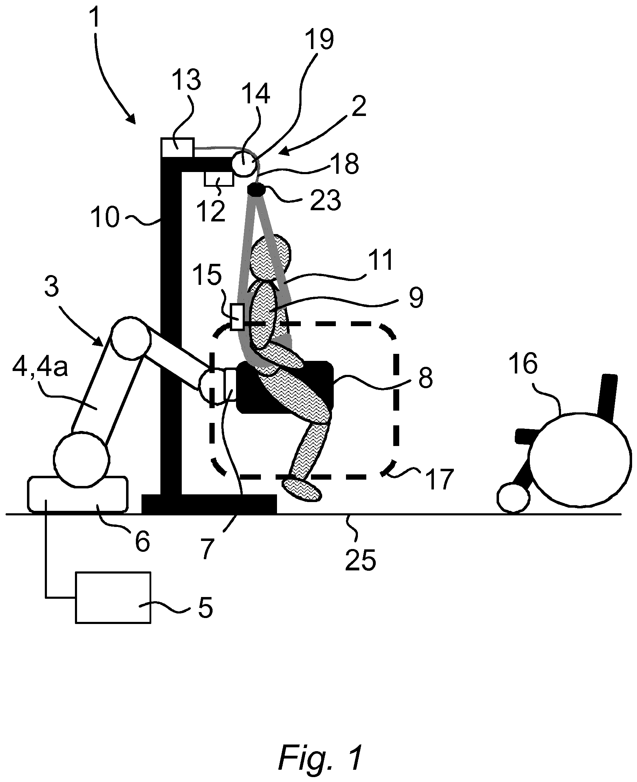

Die Fig. 1 zeigt eine erste Ausführungsform einer Hippotherapievorrichtung 1 mit einer stationären Personensicherungseinrichtung 2.

Die Hippotherapievorrichtung 1 umfasst im Falle des vorliegenden Ausführungsbeispiels außerdem einen Industrieroboter 3, der eine programmierbare Bewegungssteuerung 5 und eine automatische Bewegungsvorrichtung 4 aufweist. Die automatisehe Bewegungsvorrichtung 4 wird im Falle des vorliegenden Ausführungsbeispiels von einem Roboterarm 4a gebildet, mit mehreren über Gelenke verbundenen Gliedern. Der Roboterarm 4a weist unter anderem ein Grundgestell 6 und einen Roboterflansch 7 auf. An dem Roboterflansch 7 ist ein Sattel 8 befestigt.

Der Roboterarm 4a weist mehrere über Gelenke verbundene Glieder auf, wobei die programmierbare Bewegungssteuerung 5 eingerichtet ist, während des Betriebs die Bewegungsabläufe des Roboterarms 4a automatisch zu steuern bzw. zu regeln, um den Roboterflansch 7 des Roboterarms 4a und damit auch den Sattel 8 im Raum zu positionieren und zu bewegen.

Die Hippotherapievorrichtung 1 weist demgemäß den Sattel 8 auf, der einen Reitsitz für eine Person 9 bildet. Die Hippotherapievorrichtung 1 weist außerdem die programmierbare Bewegungssteuerung 5 und den Roboterarm 4a auf. Der Roboterarm 4a ist ausgebildet, den Sattel 8 nach einem durch die Bewegungssteuerung 5 vorgegebenen Bewegungsmuster automatisch zu bewegen.

Die Personensicherungseinrichtung 2 weist einen Grundträger 10 auf, an dem ein Körpergurt 11 verstellbar gelagert ist. Die Personensicherungseinrichtung 2 weist außerdem wenigstens einen Aktuator 12 auf, der ausgebildet ist, auf den am Grundträger 10 verstellbar gelagerten Körpergurt 11 hinsichtlich seiner räumlichen Pose automatisch einzuwirken, und eine Hilfssteuervorrichtung 13, die ausgebildet ist, den wenigstens einen Aktuator 12 in Abhängigkeit der momentanen Stellung und Bewegung des Sattels 8 und der momentanen Körperhaltung der Person 9 auf dem Sattel 8 anzusteuern.

Der Körpergurt 11 ist mittels eines Lagers 14 in wenigstens einer Richtung und/oder wenigstens einer Orientierung im Raum am Grundträger 10 verstellbar gelagert und das Lager 14 weist in einer ersten Variante eine Bremsvorrichtung auf, die ausgebildet ist, eine Bewegung, d.h. eine Drehung des Lagers 14 mit wahlweise unterschiedlichen Bremseigenschaften zu bremsen, die mittels des Aktuators 12 einstellbar sind und zwar angesteuert durch die Hilfssteuervorrichtung 13.

Der Körpergurt 11 kann aber auch mittels des Lagers 14 in wenigstens einer Richtung und/oder wenigstens einer Orientierung im Raum am Grundträger 10 schwingend gelagert sein, wobei das Lager 14 eine Schwingungsdämpfungsvorrichtung aufweist, die ausgebildet ist, eine Bewegung des Lagers 14 mit wahlweise unterschiedlichen Schwingungsdämpfungseigenschaften zu dämpfen, die mittels des Aktuators 12 einstellbar sind und zwar angesteuert durch die Hilfssteuervorrichtung 13.

Der Körpergurt 11 kann ebenso mittels einer Antriebsvorrichtung in wenigstens einer Richtung und/oder wenigstens einer Orientierung im Raum am Grundträger 10 angetrieben gelagert sein, wobei die Antriebsvorrichtung wenigstens einen Motor aufweist, der den Aktuator 12 bildet und der ausgebildet ist, den Körpergurt 11 automatisch angetrieben zu bewegen und zwar angesteuert durch die Hilfssteuervorrichtung 13.

Die Hippotherapievorrichtung 1 kann wenigstens einen Sensor 15 aufweisen, der ausgebildet ist, die räumliche Position und/oder die räumliche Orientierung des Körpers der Person 9 zu erfassen.

Im Falle des vorliegenden Ausführungsbeispiels ist der wenigstens eine Sensor 15 am Körpergurt 11 in der Nähe des Rückens der Person angeordnet.

Die Personensicherungseinrichtung 2 kann auch dazu genutzt werden, die Person 9 aus einem Rollstuhl 16 herauszuheben und auf den Sattel 8 zu setzen. Im selben Sinne kann die Personensicherungseinrichtung 2 auch dazu genutzt werden, die Person 9 aus dem Sattel 8 heraus und wieder in den Rollstuhl 16 hinein zu setzen.

Die Bewegungssteuerung 5, insbesondere der Industrieroboter 3 bzw. das mechatronische System, mit dem der Sattel 8 bzw. die Person 9 bewegt wird, kann insbesondere eine Sicherheitseinrichtung aufweisen, die mindestens zweikanalige ausgeführt sein kann.

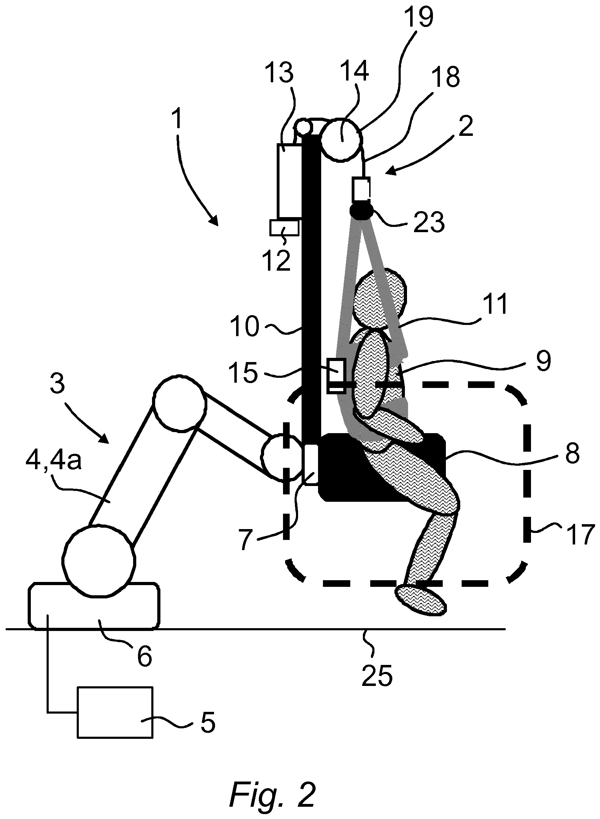

Die Sicherheitseinrichtung kann einen in sicherer Technik ausgeführten, kartesisch überwachten Arbeitsraum 17 aufweisen, der definiert sein kann durch den Einstieg und die Therapie-Höhe, -Breite, sowie -Tiefe. Die Sicherheitseinrichtung kann eine sichere kartesische Geschwindigkeit und Beschleunigung, einen sicher überwachten Arbeitsraum auf Achsebene, eine sicher überwachte Geschwindigkeit auf Achsebene umfassen. In der Fig. 1 und Fig. 2 ist der beispielhafte Arbeitsraum 17 durch ein gestrichelt dargestelltes Rechteck schematisch eingegrenzt.

Der wenigstens eine Sensor 15 kann über eine Kommunikationsverbindung mit der Hilfssteuervorrichtung 13 verbunden sein, derart, dass Informationen über die momentane Position und/oder momentane Orientierung des Körpers der Person 9 im Raum, an die Hilfssteuervorrichtung 13 übertragen wird, um die momentane Körperhaltung der Person 9 mittels der Hilfssteuervorrichtung 13 oder der Bewegungssteuerung 5 automatisch zu bestimmen.

Der Körpergurt ist im Falle der dargestellten Ausführungsbeispiele mittels eines Tragseiles 18 am Grundträger 10 aufgehängt gelagert und die Hippotherapievorrichtung 1 weist beispielsweise einen Winkelaufnehmer auf, der ausgebildet ist, die Winkelstellung des Tragseiles 18 zu erfassen.

Die Winkelstellung des Tragseiles 18 kann dabei eine erste Winkelkomponente aufweisen, welche den Auslenkungswinkel des Tragseils 18 relativ zur senkrechten Richtung definiert und eine zweite Winkelkomponente aufweisen, welche die Richtung der Auslenkung des Tragseils 18 definiert. Das Tragseil kann, wie in Fig. lund Fig. 2 dargestellt, beispielsweise über eine Seilrolle 19 längenveränderlich ausgebildet sein.

Wenn der Körpergurt 11 mittels eines längenveränderlichen Tragseiles 18 am Grundträger 10 aufgehängt gelagert ist, kann die Hippotherapievorrichtung 1 auch einen Längenaufnehmer aufweisen, der ausgebildet ist, die momentane Länge des Tragseiles 18 zu erfassen.

Die Hippotherapievorrichtung 1 kann außerdem einen Kraftaufnehmer aufweisen, der ausgebildet ist, die Zugkraft am Tragseil 18 zu erfassen.

Im Falle des Ausführungsbeispiels der Fig. 1 ist der Grundträger 10 an einem Boden 25 befestigt, an dem auch die programmierbare Bewegungsvorrichtung 4, insbesondere das Grundgestell 6 des Roboterarms 4a, befestigt ist. Die verstellbare Lagerung des Körpergurts 11 erlaubt dann eine entsprechende Anzahl von Freiheitsgraden, d.h. eine entsprechende Anzahl von Beweglichkeiten in wenigstens einer Richtung und/oder wenigstens einer Orientierung im Raum relativ zum Boden 25, insbesondere relativ zur programmierbaren Bewegungsvorrichtung 4 oder relativ zum Grundgestell 6 des Roboterarms 4a.

Der Grundträger 10 kann aber auch, wie in Fig. 2 dargestellt, an einem verstellbaren Glied der programmierbaren Bewegungsvorrichtung 4, insbesondere an dem Roboterflansch 7 des Roboterarms 4a oder an dem Sattel 8 befestigt sein, der durch die programmierbare Bewegungsvorrichtung 4 bewegt wird. Die verstellbare Lagerung des Körpergurts 11 erlaubt dann eine Anzahl von Freiheitsgraden, d.h. eine Anzahl von Beweglichkeiten in wenigstens einer Richtung und/oder wenigstens einer Orientierung im Raum relativ zum Roboterflansch 7 oder relativ zum Sattel 8.

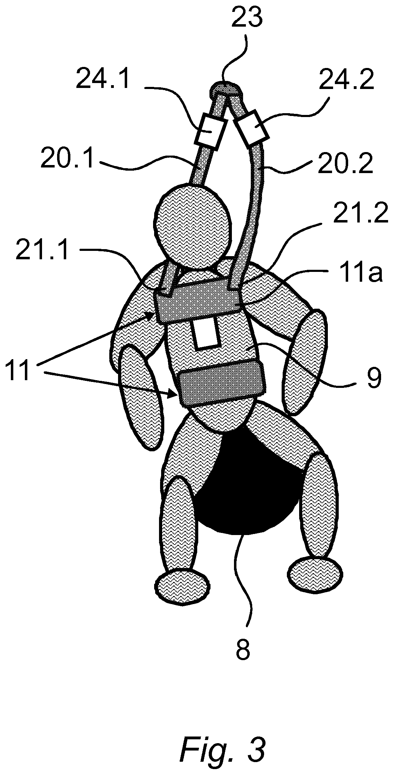

Wie insbesondere in Fig. 3 gezeigt ist, kann der Körpergurt 11 wenigstens einen ersten Traggurtabschnitt 20.1 aufweist, der einen Körpergurtabschnitt 11a des Körpergurts 11 an einer ersten Verbindungsstelle 21.1 des Körpergurtabschnitts 11a mit einer Anschlussstelle 23 des Tragseils 18 verbindet und der Körpergurt 11 kann wenigstens einen zweiten Traggurtabschnitt 20.1 aufweist, der den Körpergurtabschnitt 11a an einer von der ersten Verbindungsstelle 21.1 verschiedenen, zweiten Verbindungsstelle 21.2 des Körpergurtabschnitts 11a mit der Anschlussstelle 23 des Tragseils 18 verbindet, wobei der erste Traggurtabschnitt 20.1 einen ersten Aufnehmer 24.1 aufweist, der wenigstens eine physikalische Größe an dem ersten Traggurtabschnitt 20.1 erfasst und der zweite Traggurtabschnitt 20.2 einen zweiten Aufnehmer 24.2 aufweist, der wenigstens eine physikalische Größe an dem zweiten Traggurtabschnitt 20.2 erfasst. Die physikalische Größe kann beispielsweise eine Zugkraft in dem ersten Traggurtabschnitt 20.1 und/oder in dem zweiten Traggurtabschnitt 20.2 sein.

Die Bewegungssteuerung 5 ist in den gezeigten Ausführungsbeispielen eingerichtet, das vorgegebene Bewegungsmuster zur Ansteuerung der Bewegungsvorrichtung 4, in Abhängigkeit des Einwirkens des wenigstens einen Aktuators 12 auf den am Grundträger 10 verstellbar gelagerten Körpergurt 11, automatisch abzuändern.

## Patentansprüche

1. Hippotherapievorrichtung, aufweisend einen Sattel (8), der einen Sitz für eine Person (9) bildet, sowie eine programmierbare Bewegungssteuerung (5) und eine automatische Bewegungsvorrichtung (4), welche ausgebildet ist, den Sattel (8) nach einem durch die Bewegungssteuerung (5) auszuführenden Bewegungsmusterprogramm, das eine Abfolge an Sollwerten von Positionen und Orientierungen des Sattels (8) im Raum der Bewegungsvorrichtung (4) zur Ausführung der Bewegungsmuster vorgibt, automatisch zu bewegen, und eine Personensicherungseinrichtung (2), die einen Grundträger (10) aufweist, an dem ein Körpergurt (11) gelagert ist, wenigstens einen Sensor (15), der ausgebildet ist, wenigstens eine die Körperhaltung der Person (9) auf dem Sattel (8) kennzeichnende physikalisch Größe zu erfassen, **gekennzeichnet durch** eine Hilfssteuervorrichtung (13), die ausgebildet ist, in Abhängigkeit des Bewegungsmusterprogramms und der Position und Orientierung des Sattels (8) Erwartungswerte der physikalischen Größe, welche die erwartete Körperhaltung der Person (9) auf dem Sattel (8) kennzeichnet, heranzuziehen und mit den vom Sensor (15) erfassten, momentanen Werten der physikalischen Größe, welche die tatsächliche Körperhaltung der Person (9) auf dem Sattel (8) kennzeichnet, zu vergleichen und bei einer eine vorgegebene Toleranzschwelle überschreitenden Abweichung der momentanen Werte der physikalischen Größe von den Erwartungswerten dieser physikalischen Größe eine zugeordnete Sicherungsfunktion auszulösen, wobei die Hippotherapievorrichtung wenigstens einen Aktuator (12) umfasst, der ausgebildet ist, auf die räumliche Position und/ oder die räumliche Orientierung des Oberkörpers, des Kopfes, wenigstens eines Armes und/oder wenigstens eines Beines der Person einzuwirken, wobei die auszulösende Sicherungsfunktion ein Aktivieren des wenigstens einen Aktuators (12) umfasst.

2. Hippotherapievorrichtung nach Anspruch 1, **dadurch gekennzeichnet, dass** die die Körperhaltung der Person (9) auf dem Sattel (8) kennzeichnende physikalisch Größe die räumliche Position und/oder die räumliche Orientierung des Oberkörpers, des Kopfes, wenigstens eines Armes und/oder wenigstens eines Beines der Person (9) ist.

3. Hippotherapievorrichtung nach Anspruch 1 oder 2, **dadurch gekennzeichnet, dass** die auszulösende Sicherungsfunktion weiterhin ein Anhalten der Bewegungsvorrichtung (4) und/oder ein Ausgeben eines den unsicheren Zustand der Person (9) auf dem Sattel (8) kennzeichnenden Signals umfasst.

4. Hippotherapievorrichtung nach einem der Ansprüche 1 bis 3, **dadurch gekennzeichnet, dass** der Körpergurt (11) den wenigstens einen Sensor (15) aufweist.

5. Hippotherapievorrichtung nach einem der Ansprüche 1 bis 4, **dadurch gekennzeichnet, dass** der wenigstens eine Sensor (15) über eine Kommunikationsverbindung mit der Hilfssteuervorrichtung (13) verbunden ist, derart, dass Informationen über die momentane Position und/oder momentane Körperhaltung des Körpers der Person (9) im Raum, an die Hilfssteuervorrichtung (13) übertragen wird, um die momentane Körperhaltung der Person (9) mittels der Hilfssteuervorrichtung (13) oder der Bewegungssteuerung (5) automatisch zu bestimmen.

6. Hippotherapievorrichtung nach einem der Ansprüche 1 bis 5, **dadurch gekennzeichnet, dass** der Körpergurt (11) mittels eines Tragseiles (18) am Grundträger (10) aufgehängt gelagert ist und die Hippotherapievorrichtung (1) einen Winkelaufnehmer aufweist, der ausgebildet ist, die Winkelstellung des Tragseiles (18) zu erfassen.

7. Hippotherapievorrichtung nach einem der Ansprüche 1 bis 5, **dadurch gekennzeichnet, dass** der Körpergurt (11) mittels eines längenveränderlichen Tragseiles (18) am Grundträger (10) aufgehängt gelagert ist und die Hippotherapievorrichtung (1) einen Längenaufnehmer aufweist, der ausgebildet ist, die momentane Länge des Tragseiles (18) zu erfassen.

8. Hippotherapievorrichtung nach einem der Ansprüche 1 bis 5, **dadurch gekennzeichnet, dass** der Körpergurt (11) mittels eines Tragseiles (18) am Grundträger (10) aufgehängt gelagert ist und die Hippotherapievorrichtung (1) einen Kraftaufnehmer aufweist, der ausgebildet ist, die Zugkraft am Tragseil (18) zu erfassen.

9. Hippotherapievorrichtung nach Anspruch 4 und einem der Ansprüche 6 bis 8, **dadurch gekennzeichnet, dass** der Körpergurt (11) wenigstens einen ersten Traggurtabschnitt (20.1) aufweist, der einen Körpergurtabschnitt (11a) des Körpergurts (11) an einer ersten Verbindungsstelle (21.1) des Körpergurtabschnitts (11a) mit einer Anschlussstelle (23) des Tragseils (18) verbindet und der Körpergurt (11) wenigstens einen zweiten Traggurtabschnitt (20.2) aufweist, der den Körpergurtabschnitt (11a) an einer von der ersten Verbindungsstelle (21.1) verschiedenen, zweiten Verbindungsstelle (21.2) des Körpergurtabschnitts (11a) mit der Anschlussstelle (23) des Tragseils (18) verbindet, wobei der erste Traggurtabschnitt (20.1) einen ersten Aufnehmer (24.1) aufweist, der wenigstens eine physikalische Größe an dem ersten Traggurtabschnitt (20.1) erfasst und der zweite Traggurtabschnitt (20.2) einen zweiten Aufnehmer (24.2) aufweist, der wenigstens eine physikalische Größe an dem zweiten Traggurtabschnitt (20.2) erfasst.

10. Hippotherapievorrichtung nach einem der Ansprüche 1 bis 9, **dadurch gekennzeichnet, dass** der wenigstens eine Aktuator (12) ausgebildet ist, auf den am Grundträger (10) verstellbar gelagerten Körpergurt (11) hinsichtlich seiner räumlichen Pose automatisch einzuwirken, wobei die Hilfssteuervorrichtung (13) ausgebildet ist, den wenigstens einen Aktuator (12) in Abhängigkeit der momentanen Stellung und Bewegung des Sattels (8) und der momentanen Körperhaltung der Person (9) auf dem Sattel (8) anzusteuern.

11. Hippotherapievorrichtung nach Anspruch 10, **dadurch gekennzeichnet, dass** der Körpergurt (11) mittels eines Lagers (14) in wenigstens einer Richtung und/oder wenigstens einer Orientierung im Raum am Grundträger (10) verstellbar gelagert ist und das Lager (14) eine Bremsvorrichtung aufweist, die ausgebildet ist, eine Bewegung des Lagers (14) mit wahlweise unterschiedlichen Bremseigenschaften zu bremsen, die mittels des Aktuators (12) einstellbar sind und zwar angesteuert durch die Hilfssteuervorrichtung (13).

12. Hippotherapievorrichtung nach Anspruch 10 oder 11, **dadurch gekennzeichnet, dass** der Körpergurt (11) mittels eines Lagers (14) in wenigstens einer Richtung und/oder wenigstens einer Orientierung im Raum am Grundträger (10) schwingend gelagert ist und das Lager (14) eine Schwingungsdämpfungsvorrichtung aufweist, die ausgebildet ist, eine Bewegung des Lagers (14) mit wahlweise unterschiedlichen Schwingungsdämpfungseigenschaften zu dämpfen, die mittels des Aktuators (12) einstellbar sind und zwar angesteuert durch die Hilfssteuervorrichtung (13).

13. Hippotherapievorrichtung nach einem der Ansprüche 10 bis 12, **dadurch gekennzeichnet, dass** der Körpergurt (11) mittels einer Antriebsvorrichtung in wenigstens einer Richtung und/oder wenigstens einer Orientierung im Raum am Grundträger (10) angetrieben gelagert ist und die Antriebsvorrichtung wenigstens einen Motor aufweist, der den Aktuator (12) bildet und der ausgebildet ist, den Körpergurt (11) automatisch angetrieben zu bewegen und zwar angesteuert durch die Hilfssteuervorrichtung (13).

14. Hippotherapievorrichtung nach einem der Ansprüche 10 bis 13, **dadurch gekennzeichnet, dass** die Bewegungssteuerung (5) eingerichtet ist, das vorgegebene Bewegungsmuster zur Ansteuerung der Bewegungsvorrichtung (4), in Abhängigkeit des Einwirkens des wenigstens einen Aktuators (12) auf den am Grundträger (10) verstellbar gelagerten Körpergurt (11), automatisch abzuändern.

## Claims

1. Hippotherapy device, comprising a saddle (8) which forms a seat for a person (9), as well as a programmable movement control (5) and an automatic movement device (4) which is designed to move the saddle (8) according to one of the movement control (5) to be executed movement pattern program, which specifies a sequence of set points of positions and orientations of the saddle (8) in the space of the movement device (4) for executing the movement pattern to move automatically, and a personal security device (2), which has a base support (10), on which a body belt (11) is mounted, at least one sensor (15) which is designed to detect at least one physical variable characterizing the posture of the person (9) on the saddle (8), **characterized by** an auxiliary control device (13), which is designed as a function of the movement pattern program and the position and orientation of the saddle (8) expected values of the physical quantity, which characterizes the expected posture of the person (9) on the saddle (8), to be used and with the instantaneous values of the physical quantity recorded by the sensor (15), which the actual posture of the person (9) on the saddle (8) identifies, compares and triggers an assigned safety function in the event of a deviation of the current values of the physical variable from the expected values of this physical variable that exceeds a predetermined tolerance threshold, the hippotherapy device comprising at least one actuator (12) which is designed to target the spatial position and/or the spatial orientation of the upper body, the head, at least one arm and/or at least one leg of the person (9) to act, wherein the safety function to be triggered comprises an activation of the at least one actuator (12).

2. Hippotherapy device according to claim 1, **characterized in that** the physical position of the person (9) on the saddle (8) characterizing the spatial position and/or the spatial orientation of the upper body, the head, at least one arm and/or at least of a leg of the person (9) .

3. Hippotherapy device according to claim 1 or 2, **characterized in that** the safety function to be triggered further comprises stopping the movement device (4) and/or outputting a signal that characterizes the unsafe condition of the person (9) on the saddle (8).

4. Hippotherapy device according to one of claims 1 to 3, **characterized in that** the body belt (11) has the at least one sensor (15).

5. Hippotherapy device according to one of claims 1 to 4, **characterized in that** the at least one sensor (15) is connected via a communication link to the auxiliary control device (13), such that information about the current position and/or current posture of the body person (9) in the room is transmitted to the auxiliary control device (13) in order to automatically determine the current posture of the person (9) by means of the auxiliary control device (13) or the movement control (5) .

6. Hippotherapy device according to one of claims 1 to 5, **characterized in that** the body belt (11) is mounted suspended from the base support (10) by means of a support cable (18) and the hippotherapy device (1) has an angle sensor which is designed to control the angular position to capture the support cable (18).

7. Hippotherapy device according to one of claims 1 to 5, **characterized in that** the body belt (11) is mounted suspended from the base support (10) by means of a variable-length support cable (18) and the hippotherapy device (1) has a length transducer which is designed that to record the current length of the support cable (18) .

8. Hippotherapy device according to one of claims 1 to 5, **characterized in that** the body belt (11) is mounted suspended by means of a support cable (18) on the base support (10) and the hippotherapy device (1) has a force transducer which is designed to measure the tensile force to be recorded on the support cable (18).

9. Hippotherapy device according to claim 1 and one of claims 6 to 8, **characterized in that** the body belt (11) has at least one first support belt section (20.1) which connects a body belt section (11a) of the body belt (11) at a first connection point (21.1) of the body belt section (11a) to a connection point (23) of the support cable (18) and the body belt (11) has at least one second support belt section (20.2) which connects the body belt section (11a) to a second connection point (21.2.) that differs from the first connection point (21.1) connects the body belt section (11a) to the connection point (23) of the support cable (18), wherein the first support belt section (20.1) having a first sensor (24.1) which detects at least one physical variable on the first support belt section (20.1) and the second support belt section (20.2) has a second sensor (24.2), which detects at least one physical variable on the second support belt section (20.2).

10. Hippotherapy device according to one of claims 1 to 9, **characterized in that** the at least one actuator (12) is designed to automatically act on the body belt (11) adjustably mounted on the base support (10) with regard to its spatial pose, the auxiliary control device (13) is designed to control the at least one actuator (12) as a function of the current position and movement of the saddle (8) and the current posture of the person (9) on the saddle (8).

11. Hippotherapy device according to claim 10, **characterized in that** the body belt (11) is mounted adjustably in at least one direction and/or at least one orientation in space on the base support (10) by means of a bearing (14) and the bearing (14) has a braking device which is designed to brake a movement of the bearing (14) with optionally different braking properties, which are adjustable by means of the actuator (12) and that is controlled by the auxiliary control device (13) .

12. Hippotherapy device according to claim 10 or 11, **characterized in that** the body belt (11) is mounted to swing in at least one direction and/or at least one orientation in space on the base support (10) by means of a bearing (14) and the bearing (14) has a vibration damping device which is designed to dampen a movement of the bearing (14) with optionally different vibration damping properties which can be set by means of the actuator (12) and controlled by the auxiliary control device (13).

13. Hippotherapy device according to one of claims 10 to 12, **characterized in that** the body belt (11) is mounted driven by a drive device in at least one direction and/or at least one orientation in space on the base support (10) and the drive device has at least one motor which forms the actuator (12) and which is designed to move the body belt (11) in an automatically driven manner and that is controlled by the auxiliary control device (13).

14. Hippotherapy device according to one of claims 10 to 13, **characterized in that** the movement control (5) is set up, the predetermined movement pattern for controlling the movement device (4), depending on the action of the at least one actuator (12) on the base support (10) adjustable body belt (11) to be changed automatically.

## Revendications

1. Dispositif d'hippothérapie, comprenant une selle (8) qui forme un siège pour une personne (9), ainsi qu'une commande de mouvement programmable (5) et un dispositif de mouvement automatique (4) qui est conçu pour déplacer la selle (8) selon l'une des commandes de mouvement (5) à exécuter le programme de configuration de mouvement, qui spécifie une séquence de points de consigne de positions et d'orientations de la selle (8) dans l'espace du dispositif de mouvement (4) pour exécuter la configuration de mouvement pour se déplacer automatiquement, et un dispositif de sécurité personnel (2), qui a un support de base (10), sur lequel une ceinture corporelle (11) est montée, au moins un capteur (15) qui est conçu pour détecter au moins une variable physique caractérisant la posture de la personne (9) sur la selle (8), **caractérisée par** un dispositif de commande auxiliaire (13), qui est conçu en fonction du programme de schéma de mouvement et de la position et de l'orientation de la selle (8) valeurs attendues de la quantité physique, qui caractérise la posture attendue de la personne (9) sur la selle (8), à utiliser et avec les valeurs instantanées de la quantité physique enregistrée par le capteur (15), que la posture réelle de la personne (9) sur la selle (8) identifie, compare et déclenche une fonction de sécurité affectée en cas d'écart des valeurs courantes de la variable physique par rapport aux valeurs attendues de cette variable physique dépassant un seuil de tolérance prédéterminé, le dispositif d'hippothérapie comportant au moins un actionneur (12) qui est destiné à cibler la position spatiale et/ou l'orientation spatiale du haut du corps, de la tête, d'au moins un bras et/ou d'au moins une jambe de la personne (9) à agir, la fonction de sécurité à déclencher comprenant une activation de l'au moins un actionneur (12).

2. Dispositif d'hippothérapie selon la revendication 1, **caractérisé en ce que** la position physique de la personne (9) sur la selle (8) caractérise la position spatiale et/ou l'orientation spatiale du haut du corps, de la tête, d'au moins un bras et/ou au moins d'une jambe de la personne (9).

3. Dispositif d'hippothérapie selon la revendication 1 ou 2, **caractérisé en ce que** la fonction de sécurité à déclencher comprend en outre l'arrêt du dispositif de mouvement (4) et/ou l'émission d'un signal qui caractérise la condition dangereuse de la personne (9) sur la selle (8).

4. Dispositif d'hippothérapie selon l'une des revendications 1 à 3, **caractérisé en ce que** la ceinture corporelle (11) comporte au moins un capteur (15).

5. Dispositif d'hippothérapie selon l'une des revendications 1 à 4, **caractérisé en ce que** le au moins un capteur (15) est connecté via une liaison de communication au dispositif de commande auxiliaire (13), de telle sorte que des informations sur la position actuelle et/ou le courant la posture de la personne corporelle (9) dans la pièce est transmise au dispositif de commande auxiliaire (13) afin de déterminer automatiquement la posture actuelle de la personne (9) au moyen du dispositif de commande auxiliaire (13) ou de la commande de mouvement (5).

6. Dispositif d'hippothérapie selon l'une des revendications 1 à 5, **caractérisé en ce que** la ceinture corporelle (11) est montée suspendue au support de base (10) au moyen d'un câble de support (18) et le dispositif d'hippothérapie (1) présente un capteur d'angle qui est conçu pour contrôler la position angulaire pour capturer le câble de support (18).

7. Dispositif d'hippothérapie selon l'une des revendications 1 à 5, **caractérisé en ce que** la ceinture corporelle (11) est montée suspendue au support de base (10) au moyen d'un câble de support de longueur variable (18) et du dispositif d'hippothérapie (1) possède un transducteur de longueur conçu pour enregistrer la longueur actuelle du câble de support (18).

8. Dispositif d'hippothérapie selon l'une des revendications 1 à 5, **caractérisé en ce que** la ceinture corporelle (11) est montée suspendue au moyen d'un câble de support (18) sur le support de base (10) et le dispositif d'hippothérapie (1) présente un transducteur de force conçu pour mesurer la force de traction à enregistrer sur le câble de support (18).

9. Dispositif d'hippothérapie selon l'une des revendications 1 et l'une des revendications 6 à 8, **caractérisé en ce que** la ceinture corporelle (11) comporte au moins un premier tronçon de ceinture de soutien (20.1) qui relie un tronçon de ceinture corporelle (11a) de la ceinture corporelle (11) à un premier point de connexion (21.1) de la section de ceinture corporelle (11a) à un point de connexion (23) du câble de support (18) et la ceinture corporelle (11) a au moins une seconde section de ceinture de support (20.2) qui relie le la section de ceinture corporelle (11a) à un deuxième point de connexion (21.2) qui diffère du premier point de connexion (21.1) relie la section de ceinture corporelle (11a) au point de connexion (23) du câble de support (18), dans lequel le la première section de ceinture de support (20.1) ayant un premier capteur (24.1) qui détecte au moins une variable physique sur la première section de ceinture de support (20.1) et la seconde section de ceinture de support (20.2) a un second capteur (24.2), qui détecte en au moins une variable physique sur la seconde section de ceinture de support (20.2).

10. Dispositif d'hippothérapie selon l'une des revendications 1 à 9, **caractérisé en ce que** le au moins un actionneur (12) est conçu pour agir automatiquement sur la ceinture corporelle (11) montée de manière réglable sur le support de base (10) en ce qui concerne sa pose spatiale , le dispositif de commande auxiliaire (13) est conçu pour commander le au moins un actionneur (12) en fonction de la position et du mouvement actuels de la selle (8) et de la posture actuelle de la personne (9) sur la selle (8).

11. Dispositif d'hippothérapie selon la revendication 10, **caractérisé en ce que** la ceinture corporelle (11) est montée de manière réglable dans au moins une direction et/ou au moins une orientation dans l'espace sur le support de base (10) au moyen d'un palier (14) et le palier (14) a un dispositif de freinage qui est conçu pour freiner un mouvement du palier (14) avec des propriétés de freinage éventuellement différentes, qui sont réglables au moyen de l'actionneur (12) et qui est commandé par le dispositif de commande auxiliaire (13).

12. Dispositif d'hippothérapie selon la revendication 10 ou 11, **caractérisé en ce que** la ceinture corporelle (11) est montée oscillante dans au moins une direction et/ou au moins une orientation dans l'espace sur le support de base (10) au moyen d'un palier (14) et le palier (14) a un dispositif d'amortissement des vibrations qui est conçu pour amortir un mouvement du palier (14) avec des propriétés d'amortissement des vibrations éventuellement différentes qui peuvent être réglées au moyen de l'actionneur (12) et commandées par l'auxiliaire dispositif de commande (13).

13. Dispositif d'hippothérapie selon l'une des revendications 10 à 12, **caractérisé en ce que** la ceinture corporelle (11) est montée entraînée par un dispositif d'entraînement dans au moins une direction et/ou au moins une orientation dans l'espace sur le support de base (10) et le dispositif d'entraînement a au moins un moteur qui forme l'actionneur (12) et qui est conçu pour déplacer la ceinture corporelle (11) d'une manière entraînée automatiquement et qui est commandé par le dispositif de commande auxiliaire (13).

14. Dispositif d'hippothérapie selon l'une des revendications 10 à 13, **caractérisé en ce que** la commande de mouvement (5) est mise en place, le schéma de mouvement prédéterminé pour commander le dispositif de mouvement (4), dépendant de l'action du au moins un actionneur (12) sur le support de base (10) ceinture corporelle (11) à changer automatiquement.
